# EUROPEAN PATENT SPECIFICATION

(11) **EP 1 364 620 B1**
(45) Date of publication and mention of the grant of the patent: **19.10.2005**
(21) Application number: 03004988.6
(22) Date of filing: 11.03.2003
(51) Int. Cl.: A61B 17/64

(54) **External fixation system for treating bone fractures**
Externes Fixationssystem zur Versorgung von Knochenbrüchen
Système de fixation externe pour le traitement des fractures osseuses

(30) Priority: 20.05.2002 IT BO20020309
(43) Date of publication of application: 26.11.2003
(73) Proprietor: CITIEFFE S.r.l., 40012 Calderara di Reno (Bologna) (IT)
(72) Inventor: Mingozzi, Franco, 40012 Lippo di Calderara, Bologna (IT); Dovesi, Alan, 40100 Bologna (IT)
(74) Representative: Modiano, Guido

(56) References cited:
- DE-C- 4 102 828
- DE-U- 29 619 711
- US-A- 2 391 537
- US-A- 4 335 715
- US-A- 5 358 504

## Description

The present invention relates to an external fixation system for treating bone fractures.

Currently, known types of fixation system generally comprise supporting means for a plurality of clamps for locking the so-called pins, which are designed to be inserted in the treated bone portions to facilitate the correct formation of the bone callus between the margins of a fracture.

To facilitate the alignment of the portions of the fractured bone, some kinds of external fixation system, for example the ones particularly suitable for femoral and tibial fractures, divide the supporting means into two or more segments, which are coupled by means of hinges so that they can be orientated with respect to each other in the most appropriate directions, or by means of coupling means that allow the relative translational motion of one segment with respect to the other parallel to their longitudinal axes.

The use of external fixation systems with the characteristics described above, however, is not free from drawbacks, since relative torsional deviations are often observed between the bone portions and can compromise the correct posture and gait of the patient. DE 296 19 711 discloses a device for repositioning fractured bone fragments having a combination of features as set forth in the pre-characterizing portion of the appended claim 1.

The aim of the present invention is to obviate the cited shortcomings by providing an external fixation system which, when installed on the bone to be treated, allows to orientate the supporting means for the pin supporting clamps so as to prevent and correct relative torsional deviations between the bone portions.

Within this aim, an object of the present invention is to provide an external fixation system that can be installed easily and whose configuration can be adjusted precisely and reliably.

Another object of the present invention is to provide an external fixation system with a structure that is simple, relatively easy to provide in practice, safe in use, effective in operation and at a relatively low cost.

In accordance with the invention, there is provided an external fixation system for treating bone fractures as defined in the appended claims.

Further features will become better apparent from the detailed description of a preferred but not exclusive embodiment of an external fixation system for treating bone fractures according to the invention, illustrated only by way of non-limitative example in the accompanying drawings, wherein:
Figure 1 is a perspective view of the external fixation system according to the invention, applied to a fractured long bone;
Figure 2 is a partially sectional side elevation view of the means for connection between the first support and the second support of the fixation system;
Figure 3 is a perspective view of a constructive detail of the connection means;
Figure 4 is a sectional front view of further constructive details of the connection means.

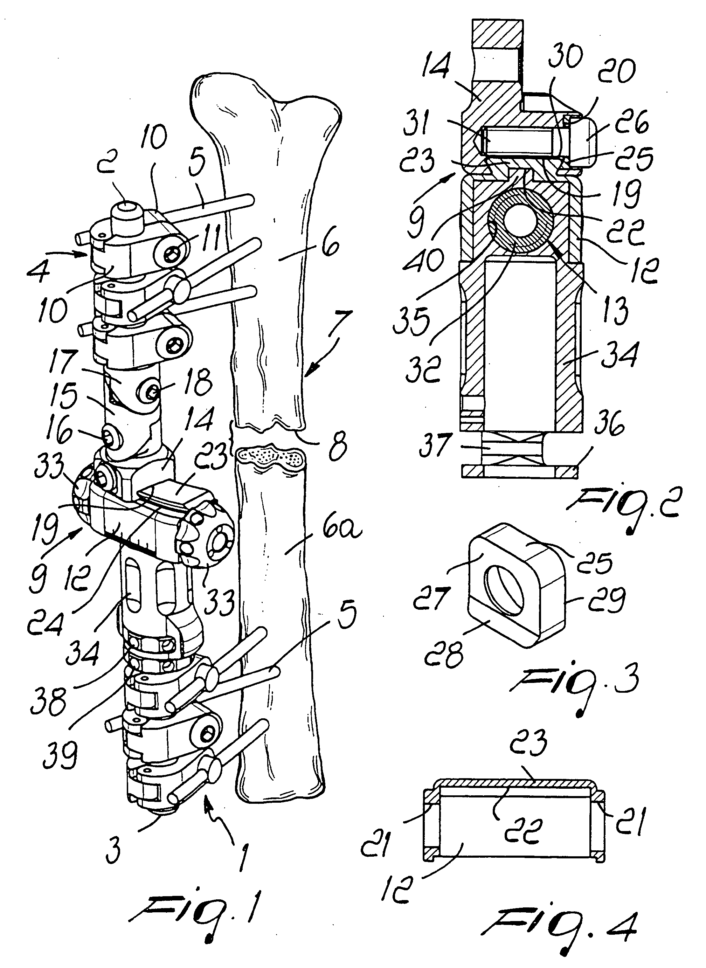

With particular reference to Figure 1, the reference numeral 1 generally designates an external fixation system for treating bone fractures, according to the invention.

The fixation system comprises a first support 2 and a second support 3, both of which are substantially shaped like a cylindrical rod, respectively for first and second pluralities of clamps 4 for locking pins 5 to be inserted in portions 6, 6a of bone, in the specific case of a long bone 7, formed by a fracture edge 8. Connection means 9 are interposed between the first and second supports 2 and 3 and allow adjustable relative rotation between the first support 2 and the second support 3 about an axis that substantially coincides with a longitudinal axis of the bone 7.

Each one of the clamps 4 is constituted, in a known manner, by a pair of jaws 10, which are articulated at one end and are fastened on the respective support 2 and 3 by a transverse screw 11 that is arranged at the other end; the end portion of the stem of the transverse screw 11 is perforated in order to allow the passage of the corresponding pin 5. In this manner it is possible to orientate the clamp 4 and therefore the pin 5 in the intended direction, so as to thread said pin into the bone 7 according to the most appropriate configuration.

The connection means 9 comprise, according to the invention, a guiding head 12 that is connected adjustably, by means of screw means 13, to the second support 3; a slider 14 is slidingly coupled to said head and is rigidly coupled to the first support 2, which preferably can be orientated with respect to the slider 14 along two perpendicular rotation axes, since it is constituted by a first segment 15, which is pivoted to the slider 14 with a first pivot 16, and by a second segment 17, which is articulated to the first segment 15 by means of a second pivot 18 that is perpendicular to the first pivot 16.

The slider 14 is affected, in a downward region, by a slot 19 that has a dovetail transverse cross-section and lies, with its centerline, along a circular arc which, when the fixation system is installed on the bone 7, is centered substantially on the diaphyseal longitudinal axis of said bone.

The slider 14 further has a lateral opening 20 that is connected to the slot 19 (Figure 2).

The guiding head 12 (Figure 4) has a substantially parallelepipedal box-like shape that is open in a downward region and is affected by a transverse through hole 21; a longitudinal groove 22 having a substantially rectangular transverse cross-section is formed inside the head 12. The head 12 has, in an upward region, a raised portion 23 that has a dovetail transverse cross-section that is complementary to the cross-section of the slot 19 and is engaged along it, forming two side walls 24: the raised portion 23 lies with its centerline along a circular arc which, when the fixation system is installed on the bone 7, is substantially centered on the diaphyseal longitudinal axis of said bone.

The mutual locking of the slider 14 and the guiding head 12, and accordingly between the first support 2 and the second support 3, is provided by means of a shaped washer 25, shown in Figure 3, which is accommodated in the opening 20 of the slider 14 and is locked against a side 24 of the raised portion 23 by a locking screw 26 that is threaded into the slider 14. The shaped washer 25 is substantially quadrangular and has, on a first face 27, a wedge-like protrusion 28 that is suitable to engage under the side 24 of the raised portion 23; on the second face 29 there is instead a circular through seat 30 that has two diameters, in order to allow the passage of the stem 31 of the locking screw 26 and its flush insertion. The washer 25 is suitable to fasten the slider 14 against the raised portion 23 in a preset angular position, eliminating the coupling plays.

The screw means 13 that connect the guiding head 12 to the second support 3 comprise a threaded bar 32, which is internally hollow and is engaged in the transverse through hole 21 and is locked thereat by a pair of lateral rings 33; the screw means 13 further comprise a sleeve 34, which is provided in an upward region with a transverse female thread 35 in which the bar 32 is screwed, and is connected in a downward region and coaxially to the second support 3: there is in fact a ring 36, which is connected coaxially to the lower edge of the sleeve 34 by means of a bridge 37, that constitutes an abutment for an axial ring 38 and a complementary axial ring 39, which are screwed onto a threaded end portion of the second support 3. The ring 38 and the complementary ring 39 are suitable to adjust precisely the axial position of the second support 3 with respect to said sleeve 34.

At the top of the sleeve there is a ridge 40, which has a substantially rectangular transverse cross-section and is suitable to engage along the groove 22 of the guiding head 12: by means of the screw 13, the sleeve 34 and accordingly the second support 3 are guided slidingly and adjustably parallel to their axis along the head 12 in order to determine, with the lateral rings 33, the most suitable configuration for the external fixation of the portions 6, 6a of the bone 7.

The fixation system further comprises strain-gauge means, which are inserted in the threaded bar 32 in order to measure the deformation of said fixation system and of the bone 7 during the formation of the bone callus at the fracture edge 8.

The method of use of the external fixation system according to the invention is as follows: the adjustment of the mutual position of the first and second supports 2 and 3 provided with the clamps 4 for locking the pins 5 inserted in the portions 6, 6a of the bone 7 can be performed with multiple degrees of freedom, i.e., by orientating the first support 2 with respect to the slider 14 along two perpendicular axes formed respectively by the first pivot 16 and by the second pivot 18, by making the second support 3 and the sleeve 34 perform a translational motion with respect to the head 12 parallel to its axis, by acting on the lateral rings 33 and performing an axial translational motion of the second support 3 with respect to the sleeve by acting on the axial ring and complementary ring 38 and 39. Furthermore, it is possible to produce a relative rotation between the first support 2 and the second support 3 about an axis which, when the fixation system is installed, substantially coincides with the longitudinal diaphyseal axis of the bone 7: the slider 14 can in fact slide along the raised portion 23 of the guiding head 12 and can be locked in the optimum position by tightening the shaped washer 25 by means of the corresponding locking screw 26, on the side 24 of the raised portion 23.

The external fixation system according to the invention is certainly effective and precise in resetting and locking the portions 6, 6a of the bone 7 formed by a fracture edge 8, particularly when it is necessary to correct any torsional deviations that have occurred along the longitudinal axis of the bone. The lockable dovetail coupling between the guiding head 12 and the slider 14, ensured by the shaped washer 25, makes the fixation system particularly rigid, eliminating plays and generating contact forces that tend to fasten said slider 14 on the head 12.

It has thus been shown that the invention achieves the intended aim and objects.

The invention thus conceived is susceptible of numerous modifications and variations within the scope of the appended claims.

In practice, the materials used, as well as the shapes and the dimensions, may be any according to requirements without thereby abandoning the scope of the protection of the appended claims.

Where technical features mentioned in any claim are followed by reference signs, those reference signs have been included for the sole purpose of increasing the intelligibility of the claims and accordingly such reference signs do not have any limiting effect on the scope of each element identified by way of example by such reference signs.

## Claims

1. An external fixation system for treating bone fractures, comprising a first support (2) and a second support (3) respectively for first and second series of locking clamps (4) for pins (5) to be inserted in the portions (6, 6a) of bone (7) formed by a fracture edge (8), and connection means (9) interposed between said first and second supports (2, 3), which allow adjustable relative rotation between said first and second supports about an axis that substantially coincides with a longitudinal axis of said bone (7), said connection means (9) comprising a guiding head (12) that is adjustably connected by screw means (13) to said second support (3), said head (12) being slidingly coupled to a slider (14) that is coupled to said first support (2) so that it can be orientated along two perpendicular axes, **characterized in that** said guiding head (12) has, in an upward region, a transverse raised portion (23) that is substantially dovetail-shaped and **in that** said slider (14) is affected, in a downward region, by a slot (19) whose transverse cross-section is substantially complementary to the cross-section of said raised portion (23), said raised portion (23) and said slot (19) being arranged along a circular arc centered on said longitudinal axis of said bone (7), said slider (14) having a lateral opening (20) that is connected to said slot (19) for accommodating a shaped washer (25) that can be locked on one of the sides (24) of said raised portion (23) by means of a locking screw (26) that is threaded into said slider (14), said washer (25) being adapted to fasten said slider (14) against said raised portion (23) in a preset angular position.

2. The external fixation system according to claim 1, **characterized in that** said shaped washer (25) is substantially quadrangular and has, on a first face (27), a wedge-shaped raised portion (28) that is adapted to engage under said side (24) of said raised portion (23) and is affected on the second face (29) by a circular through seat (30) for said locking screw (26).

3. The external fixation system according to claim 1, **characterized in that** said guiding head (12) has a substantially parallelepipedal box-like shape that is open in a downward region and is affected by a transverse through hole (21), said screw means (13) comprising an internally hollow threaded bar (32) that is engaged in said through hole (21) and is locked by a pair of lateral rings (33), and a sleeve (34), which is provided in an upward region with a transverse female thread (35), in which said bar (32) is screwed, and is connected in a downward region to said second support (3) by means of an axial ring (38) and an axial complementary ring (39) which are screwed onto a threaded end portion of said second support (3), so that said second support is guided so that it can slide adjustably parallel to its own axis along said head (12).

## Patentansprüche

1. Externes Fixationssystem zur Behandlung von Knochenbrüchen umfassend einen ersten Träger (2) und einen zweiten Träger (3) jeweils für erste und zweite Serien von Feststellhalterungen (4) für Stifte (5), die in die Teile (6, 6a) von Knochen (7) eingesetzt werden sollen, die von einer Bruchkante (8) gebildet sind, und Verbindungsmittel (9), die zwischen die ersten und zweiten Träger (2, 3) eingesetzt sind, die einstellbar relative Rotation zwischen den ersten und zweiten Trägern um eine Achse ermöglichen, die im Wesentlichen mit einer Längsachse des Knochens (7) zusammenfällt, wobei die Verbindungsmittel (9) einen Führungskopf (12) umfassen, der einstellbar durch Schraubenmittel (13) mit dem zweiten Träger (3) verbunden ist, wobei der Kopf (12) verschiebbar mit einem Gleiter (14) gekoppelt ist, der mit dem ersten Träger (2) gekoppelt ist, so dass er entlang zweier senkrechter Achsen ausgerichtet werden kann, **dadurch gekennzeichnet, dass** der Führungskopf (12) in einem oberen Bereich einen quergerichteten erhabenen Teil (23) aufweist, der im Wesentlichen schwalbenschwanzförmig ist und **dadurch**, dass der Gleiter (14) in einem unteren Bereich mit einem Schlitz (19) versehen ist, dessen Querschnitt im Wesentlichen zum Querschnitt des erhabenen Teils (23) komplementär ist, wobei der erhabene Teil (23) und der Schlitz (19) entlang eines Kreisbogens angeordnet sind, der auf der Längsachse des Knochens (7) zentriert ist, wobei der Gleiter (14) eine seitliche Öffnung (20) aufweist, die mit dem Schlitz (19) verbunden ist, um eine Formscheibe (25) aufzunehmen, die an einer der Seiten (24) des erhabenen Teils (23) mittels einer Feststellschraube (26) festgelegt werden kann, die in den Gleiter (14) eingeschraubt wird, wobei die Scheibe (25) geeignet ist, den Gleiter (14) gegen den erhabenen Teil (23) in einer bestimmten Winkelposition zu befestigen.

2. Externes Fixationssystem nach Anspruch 1 , **dadurch gekennzeichnet, dass** die Formscheibe (25) im Wesentlichen viereckig ist und auf einer ersten Seite (27) einen keilförmigen erhabenen Teil (28) aufweist, der geeignet ist, unter der Seite (24) des erhabenen Teils (23) einzugreifen, und auf der zweiten Seite (29) mit einem kreisförmigen durchgehenden Sitz (30) für die Feststellschraube (26) versehen ist.

3. Externes Fixationssystem nach Anspruch 1, **dadurch gekennzeichnet, dass** der Führungskopf (12) eine im Wesentlichen kastenartige Form eines Parallelepipeds aufweist, die in einem unteren Bereich offen ist und mit einem quergerichteten Durchtritt (21) versehen ist, wobei die Schraubenmittel (13) eine innen hohle Gewindestange (32) umfassen, die in den Durchtritt (21) eingreift und durch ein Paar seitlicher Ringe (33) festgelegt ist, und eine Hülse (34), die in einem oberen Bereich mit einem quergerichteten Innengewinde (35) versehen ist, in das die Stange (32) eingeschraubt wird, und in einem unteren Bereich mit dem zweiten Träger (3) mittels eines axialen Rings (38) verbunden ist und einem axialen komplementären Ring (39), die auf einen mit Gewinde versehenen Endteil des zweiten Trägers (3) aufgeschraubt werden, so dass der zweite Träger geführt ist, so dass er sich einstellbar parallel zu seiner eigenen Achse entlang des Kopfes (12) verschieben kann.

## Revendications

1. Système de fixation externe pour le traitement de fractures osseuses, comprenant un premier support (2) et un second support (3) respectivement pour des première et seconde séries de brides de verrouillage (4) pour des broches (5) à insérer dans les parties (6, 6a) d'os (7) formées par une ligne de fracture (8), et des moyens de liaison (9) disposés entre lesdits premier et second supports (2, 3) qui permettent une rotation relative réglable entre lesdits premier et second supports autour d'un axe qui coïncide sensiblement à l'axe longitudinal dudit os (7), lesdits moyens de liaison (9) comprenant une tête de guidage (12) qui est reliée de façon réglable par des moyens à vis (13) par rapport audit second support (3), ladite tête (12) étant assemblée de façon coulissante à un coulisseau (14) qui est assemblé audit premier support (2) de sorte qu'il peut être orienté le long de deux axes perpendiculaires,
**caractérisé en ce que** ladite tête de guidage (12) présente, dans une région vers le haut, une partie saillante transversale (23) qui est sensiblement en forme de queue d'aronde et **en ce que** ledit coulisseau (14) présente, dans une région vers le bas, une fente (19) dont la coupe transversale est sensiblement complémentaire de la coupe transversale de ladite partie saillante (23), ladite partie saillante (23) et ladite fente (19) étant agencées le long d'un arc circulaire centré sur ledit axe longitudinal dudit os (7), ledit coulisseau (14) ayant une ouverture latérale (20) qui est reliée à ladite fente (19) pour pouvoir recevoir une rondelle façonnée (25) qui peut être verrouillée sur l'un des côtés (24) de ladite partie saillante (23) par l'intermédiaire d'une vis de verrouillage (26) qui est vissée dans ledit coulisseau (14), ladite rondelle (25) étant adaptée pour fixer ledit coulisseau (14) contre ladite partie saillante (23) selon une position angulaire prédéterminée.

2. Système de fixation externe selon la revendication 1,
**caractérisé en ce que** ladite rondelle façonnée (25) est sensiblement quatrangulaire et présente, sur une première face (27), une partie saillante en forme de coin (28) qui est adaptée pour s'engager sous ledit côté (24) de ladite partie saillante (23) et est pourvue, sur la seconde face (29), d'une portée circulaire traversante (30) pour ladite vis de verrouillage (26).

3. Système de fixation externe selon la revendication 1,
**caractérisé en ce que** ladite tête de guidage (12) a une forme de boîte sensiblement parallélépipédique qui est ouverte dans une région vers le bas et présente un trou traversant transversal (21), lesdits moyens à vis (13) comprenant une tige filetée intérieurement creuse (32) qui est engagée dans ledit trou traversant (21) et est verrouillée par une paire de bagues latérales (33), et un manchon (34), qui est muni, dans une région vers le haut, d'un taraudage transversale (35), dans lequel ladite tige (32) est vissée, et est relié dans une région vers le bas audit second support (3) par l'intermédiaire d'une bague axiale (38) et d'une bague axiale complémentaire (39) qui sont vissées sur une partie d'extrémité filetée dudit second support (3), de sorte que ledit second support est guidé si bien qu'il peut coulisser, de façon réglable, parallèlement à son propre axe le long de ladite tête (12).
